# EUROPEAN PATENT SPECIFICATION

(11) **EP 1 786 771 B1**
(45) Date of publication and mention of the grant of the patent: **24.11.2010**
(21) Application number: 05773802.3
(22) Date of filing: 26.08.2005
(51) Int. Cl.: C07D 207/40

(54) **NOVEL POLYMORPHS OF THE POTASSIUM SALT OF ATORVASTATIN**
NEUE POLYMORPHE AUS DEM KALIUMSALZ VON ATORVASTATIN
NOUVEAUX POLYMORPHES DU SEL DE POTASSIUM DE L'ATORVASTATINE

(30) Priority: 27.08.2004 DK 200401293
(43) Date of publication of application: 23.05.2007
(73) Proprietor: Sandoz A/S, 5220 Odense SØ (DK)
(72) Inventor: BECK, Frederik, Barfoed, DK-2880 Bagsvaerd (DK); FISCHER, Erik, DK-3500 Værløse (DK)
(74) Representative: Klinge, Ulla Callesen
(86) International application number: PCT/DK2005/000544
(87) International publication number: WO 2006/021216

(56) References cited:
- EP-A- 0 409 281

## Description

### Technical Field

The present invention relates to novel polymorphs of the potassium salt of the compound Atorvastatin, the chemical name of which is (βR,δR)-2-(p-fluorophenyl)-β,δ-dihydroxy-5-isopropyl-3-phenyl-4-(phenylcarbamoyl)pyrrole-1-heptanoic acid. The novel polymorphs according to the invention are white crystalline or amorphous solids with characteristic melting points and X-ray powder diffraction patterns, said polymorphs overcoming many of the disadvantages of the Atorvastatin salts known so far.

### Background Art

Atorvastatin or (βR,δR)-2-(p-fluorophenyl)-β,δ-dihydroxy-5-isopropyl-3-phenyl-4-(phenylcarbamoyl)pyrrole-1-heptanoic acid having the structural formula 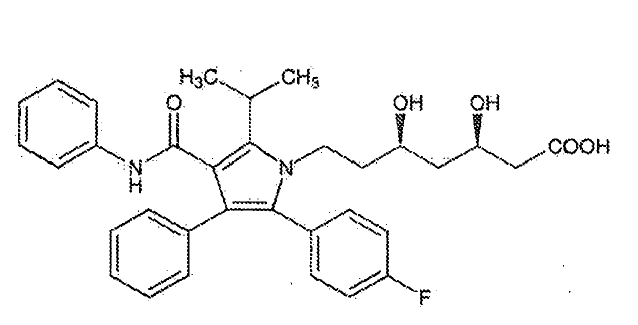 is a compound which can be successfully used for the lowering of the cholesterol level in plasma. The compound as the free acid is developed by Warner-Lambert Company and covered by EP 0 247 633 B1, where Example 2 specifically concerns the sodium salt of Atorvastatin. The calcium trihydrate of Atorvastatin has been made the subject of the supplementary protection certificate (SPC) CR 1997 00044 in Denmark, where the compound is marketed as a pharmaceutical named Zarator®. This pharmaceutical acts as a selective inhibitor of the enzyme 3-hydroxy-3-methylglutaryl-coenzyme A reductase (HMG-CoA reductase), which is the rate determining enzyme in the cholesterol synthesis. The compound increases the formation of LDL (low density lipoproteins) receptors, thereby i.a. lowering the level of LDL cholesterol in the blood.

The hemicalcium salt of the R(R*,R*) stereoisomer of Atorvastatin is disclosed in EP 0 409 281 B1. A divisional thereof, published as EP 1 061 073 A1, claims the monosodium, monopotassium, N-methylglucamine, hemimagnesium and hemizinc salts of the R(R*,R*) stereoisomer. However, none of these documents report any physical characteristics of the potassium salt of Atorvastatin. Neither do these two documents offer any other information, be it in the description or in the examples which might be used to establish the physical characteristics.

WO 03/068739 discloses a method of manufacturing an amorphous form of the hemi-calcium salt of Atorvastatin, wherein Atorvastatin or an alkali metal or ammonium salt thereof is converted to the hemi-calcium salt. No physical characteristics of any potassium salt of Atorvastatin are provided.

Amine salts of Atorvastatin and other statins are known from WO00/171 A1. These amine salts are used for the purification of Atorvastatin. In addition, salts of Atorvastatin with the amino acids lysine, arginine and ornithine are known from WO03/082816 A1. It is stated in said publication that these amino acid salts exhibit an improved bioavailability over the crystalline calcium salt of Atorvastatin.

The earliest attempts to prepare Atorvastatin have led to the compound in amorphous form. According to the more recent approaches reported in EP 0 848 704 B1 and EP 0 848 705 B1, concerning novel polymorphous forms of the hemicalcium salt of Atorvastatin, the original amorphous form of Atorvastatin had unsuitable filtration and drying characteristics and must be protected from heat, light, oxygen and moisture.

An attempt to solve this problem is presented in EP 0 848 704 B1 and EP 0 848 705 B1, which, as mentioned above, describe the preparation of novel polymorphous forms of the hemicalcium salt of Atorvastatin. According to EP 0 680 320 B1, an attempt has also been made to stabilise the calcium salt of Atorvastatin by adding a basic Ca, Mg or Li salt.

A need still exists for polymorphs of Atorvastatin which have suitable filtration and drying characteristics and which exhibit superior solubility characteristics at the relevant pH-range in the gastro-intestinal tract compared to the calcium salt of Atorvastatin.

### Disclosure of Invention

According to the present invention, hitherto unknown polymorphs of the potassium salt of Atorvastatin displaying unexpected improved solubility characteristics compared to calcium Atorvastatin are provided. One of said polymorphs is obtained directly by precipitation of the potassium salt of Atorvastatin in ethanol, i.e. polymorph Form I. Precipitation from 1-propanol yields amorphous potassium Atorvastatin, i.e. polymorph Form II, whereas recrystallising polymorph form I from 2-propanol yields polymorph Form III.

Another aspect of the present invention is a method of preparing any of the novel polymorphs.

A further aspect is a pharmaceutical formulation comprising as active ingredient a member selected from potassium Atorvastatin, Form I, potassium Atorvastatin, Form II and potassium Atorvastatin, Form III, together with conventional pharmaceutically acceptable excipients, diluents, carriers and/or additives.

### Brief Description of the Drawing(s)

The invention is explained in detail below with reference to the drawings, in which
Fig. 1 is a thermogram of potassium Atorvastatin, Form I, made by DSC analysis of the compound,
Fig. 2 is a thermogram of potassium Atorvastatin, Form II, made by DSC analysis of the compound,

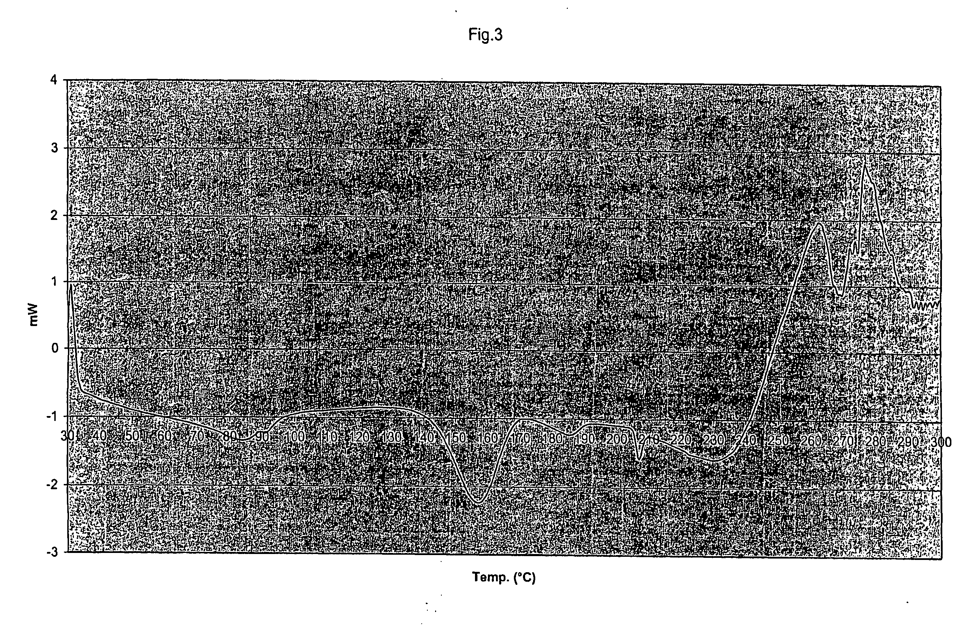

Fig. 3 is a thermogram of potassium Atorvastatin, Form III, made by DSC analysis of the compound,
Fig. 4 is an X-ray powder diffraction pattern of potassium Atorvastatin, Form I,
Fig. 5 is an X-ray powder diffraction pattern of potassium Atorvastatin, Form II, and
Fig. 6 is X-ray powder diffraction pattern of potassium Atorvastatin, Form III.

### Best Mode(s) for Carrying out the Invention

The novel polymorphs are white crystalline (Form I and III) or amorphous (Form II) solids with characteristic melting points and X-ray powder diffraction patterns.

The preparation of the polymorphs of the invention as well as their physical data will appear more clearly from the examples below.

Potassium Atorvastatin Form I is obtained by a method comprising the following steps: i) dissolution of Atorvastatin free acid in ethanol, ii) addition of potassium hydroxide, and iii) isolation of the precipitated potassium Atorvastatin polymorph Form I obtained by filtration.

In the above method potassium hydroxide is added in at least a molar ratio to Atorvastatin. A suitable Atorvastatin : KOH ratio is in the range 1 : 1, more preferably 1 : 1.1 - 1.5.

Potassium Atorvastatin Form II is obtained by a method comprising the following steps: i) dissolution of Atorvastatin free acid in 1-propanol, ii) addition of potassium hydroxide, and iii) isolation of the precipitated potassium Atorvastatin polymorph Form II obtained by filtration.

In the above method potassium hydroxide is added in at least a molar ratio to Atorvastatin. A suitable Atorvastatin : KOH ratio is in the range 1 : 1, more preferably 1 : 1.1 - 1.5.

Potassium Atorvastatin Form III is obtained by recrystallisation of potassium Atorvastatin Form I with 2-propanol. Thus potassium Atorvastatin Form III is obtained by a method comprising the following steps: i) providing potassium Atorvastatin polymorph Form I, ii) dissolution thereof in 2-propanol, and iii) isolation of the precipitated potassium Atorvastatin Form III obtained by filtration.

The novel polymorphs may be obtained in the form of a hydrate or solvate thereof. Suitable hydrates or solvates may be obtained from solvents selected from the group comprising water, alcohols, organic acids and bases, nitriles, ketones, ethers and (optionally halogenated) hydrocarbons.

As non-limiting examples of the above solvents, mention may be made of water, ethanol, 1-propanol and 2-propanol, acetic acid, pyridine, acetonitrile, acetone, tetrahydrofuran, chloroform and toluene.

The novel polymorphs of the invention can be formulated and administered in a wide variety of oral and parenteral dosage forms. Thus, the formulations of the present invention can be administered by injection, that is, intravenously, intramuscularly, intracutaneously, subcutaneously, intraduodenally or intraperitoneally. Also, the polymorphs of the present invention can be administered by inhalation, for example, intranasally. Additionally, the polymorphs of the present invention can be administered transdermally.

For preparing pharmaceutical formulations from the polymorphs of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, solubilizers, lubricants, suspending agents, binders, preservatives, tablet disintegrating agents, or an encapsulating material.

The pharmaceutical formulation is preferably in unit dosage form. In such form, the formulation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of active component in a unit dose formulation may be varied or adjusted from 0.5 mg to 100 mg, preferably 2.5 mg to 80 mg according to the particular application. Particularly preferred unit doses contain 10, 20, 40 or 80 mg, calculated as the free acid, corresponding to 10.76, 21.52, 43.04 and 86.08 of potassium Atorvastatin, respectively. The formulation can, if desired, also contain other compatible therapeutic agents.

### EXAMPLES

### Example 1

### Synthesis of crystalline potassium Atorvastatin, Form I

Atorvastatin as the free acid (186.8 g; 0.33 mmol) was dissolved in ethanol (1.5 L) in a three necked round-bottomed flask (volume 3 L) provided with mechanical stirring and a CaCl₂ tube. Powdered potassium hydroxide (24.3 g; 0.37 mol; 1.1 eq) was added under vigorous stirring over a period of 5 minutes, whereafter the reaction mixture was stirred overnight.

The colourless precipitate was isolated by filtration and washed on the filter using cold ethanol (200 mL) followed by drying under vacuum at 45 °C for 18 hours. The material was analysed by microanalysis:

| | | | |
|---|---|---|---|
| Calculated: | C 66.42 | H 5.74 | N 4.69 |
| Found: | C 64.05 | H 5.71 | N 4.42 |

Karl Fischer titration showed a water content of 2.64 % in the sample, corresponding to a monohydrate. When correcting for the water content the values were:

| | | | |
|---|---|---|---|
| Calculated: | C 64.67 | H 5.89 | N 4.57 |

HPLC analysis data:

| | |
|---|---|
| Column: | Nucleosil 10 C18, 5 µm, 4.6 x 250 mm. |
| Flow: | 0.6 mL/min. |
| Wavelength: | 230 nm |
| Mobile phase: | Methanol/buffer pH 7 90: 10 (buffer pH 7 was deionised water (950 mL) mixed with triethylamine (5.5 mL); conc. phosphoric acid 85 % was added to pH 7) |
| Sample conc.: | 0.02 % (w/w) in mobile phase |
| RT: | ca. 4.1 min. |

The HPLC analysis showed a purity of 100 %.

The potassium Atorvastatin Form I was analysed by DSC analysis and X-ray powder diffractography.

The DSC analysis was performed by analysing a sample of approximately 2 mg by linear heating from 30 °C to 300 °C in a Mettler DSC 822 apparatus.

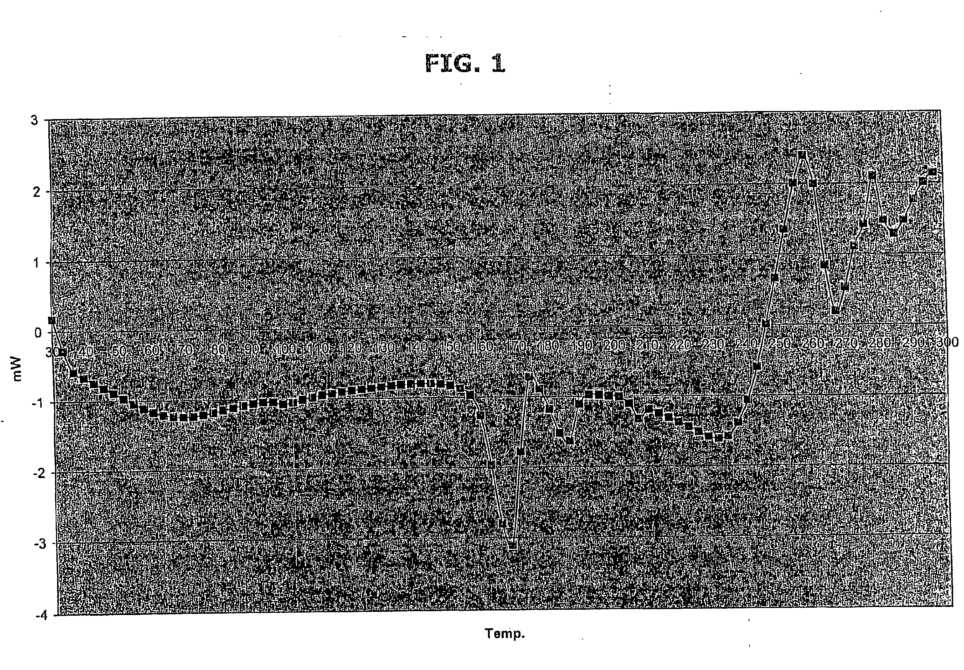

The melting point by DSC analysis was 155-165 °C (See Fig. 1).

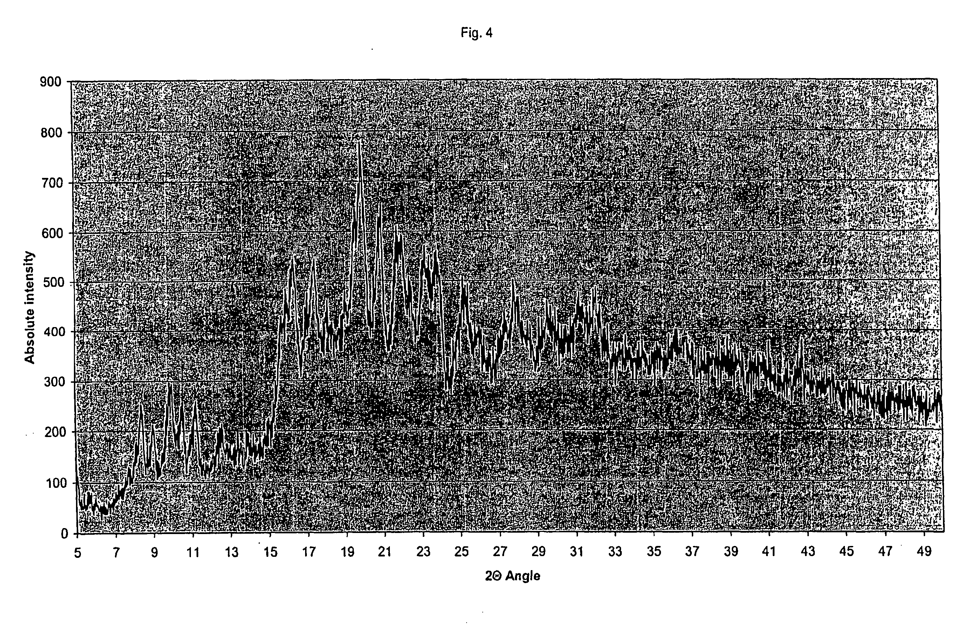

The X-ray powder diffractogram was performed on a Philips PW 3710 diffractometer using CuK_{α} radiation, wavelengths: (A): 1.54060/1.54443 and an exposure time of 2h 3 min. The X-ray powder diffractogram for potassium Atorvastatin, Form I, presented as Fig. 4, showed a crystalline solid. Said pattern shows characteristic peaks at 2θ values of 19.92, 20.98 and 22.08. More particularly, polymorphs Form I showed the following characteristic 2θ angle values:

| **2**Θ **angle** |
|---|
| 8.4 |
| 9.0 |
| 10.0 |
| 10.5 |
| 11.24 |
| 16.38 |
| 17.46 |
| 18.12 |
| **19.92** |
| **20.98** |
| **22.08** |
| 23.24 |
| 23.84 |
| 25.2 |
| 27.8 |
| 29.6 |
| 31.3 |
| 32.16 |
| 36.34 |
| 42.8 |

### Example 2

### Synthesis of amorphous potassium Atorvastatin, polymorph II

Atorvastatin as the free acid (1.0 g; 0.18 mmol) was dissolved in 1-propanol (10 mL) in a round-bottomed flask (volume 50 mL) provided with mechanical stirring and a CaCl₂ tube. Powdered potassium hydroxide (0.1g; 1.8 mmol; 1.1 eq) was added under vigorous stirring over a period of 5 minutes, and the reaction mixture was stirred overnight.

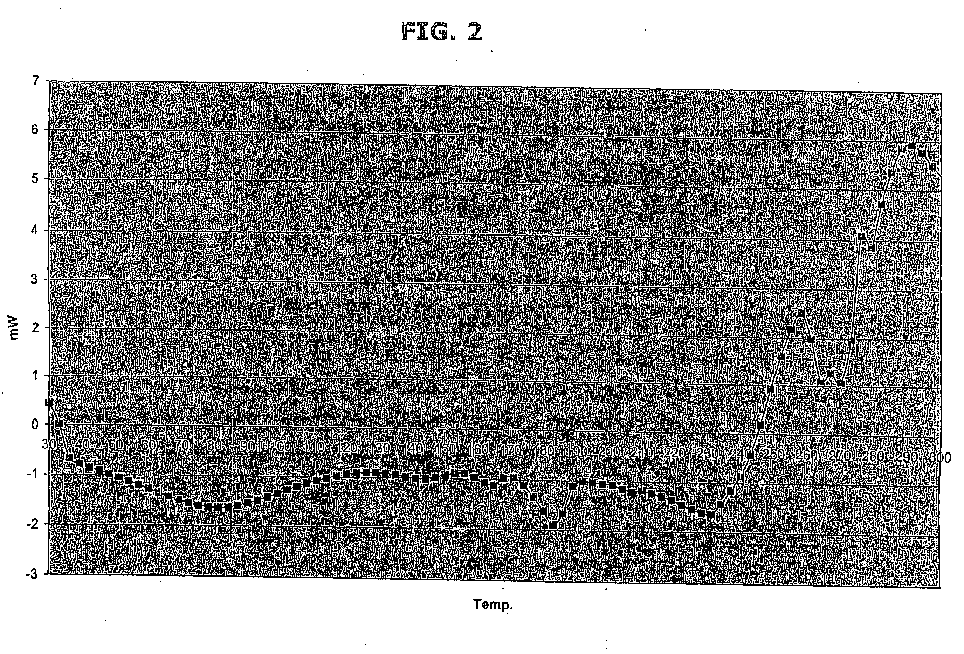

The colourless precipitate was isolated by filtration and washed on the filter using cold 1-propanol (3 mL) followed by drying under vacuum at 45 °C for 18 hours. The material was analysed by HPLC analysis, which gave a purity of 100 % using the same HPLC analysis data as in example 1. The melting point by DSC analysis was 173-183 °C (See Fig. 2).

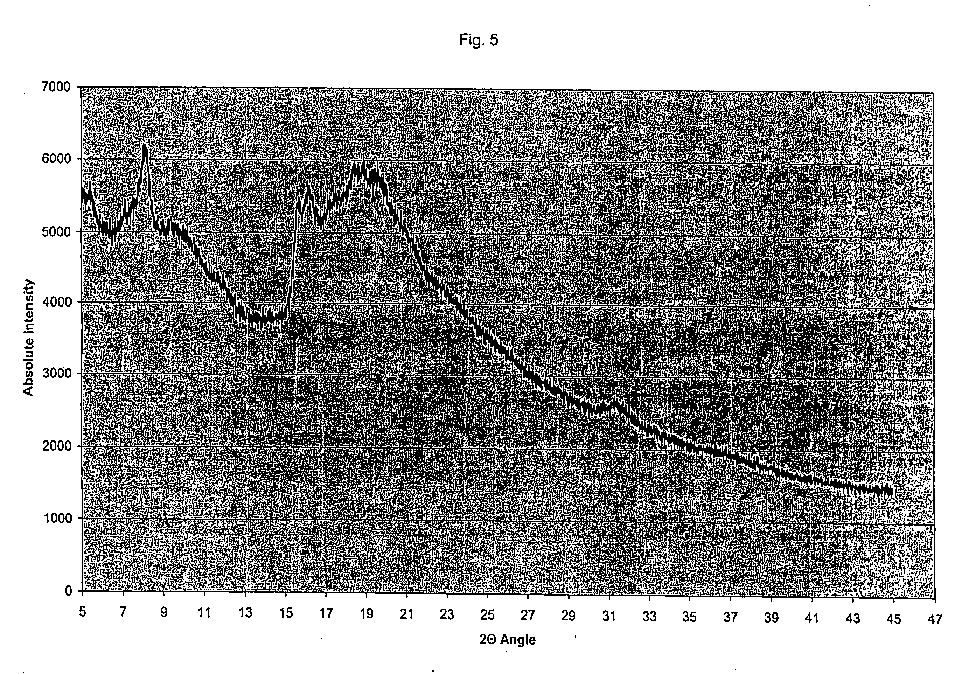

The X-ray powder diffractogram, obtained as in Example 1 above and presented as Fig. 5, showed an amorphous substance.

### Example 3

### Synthesis of crystalline potassium Atorvastatin, polymorph III

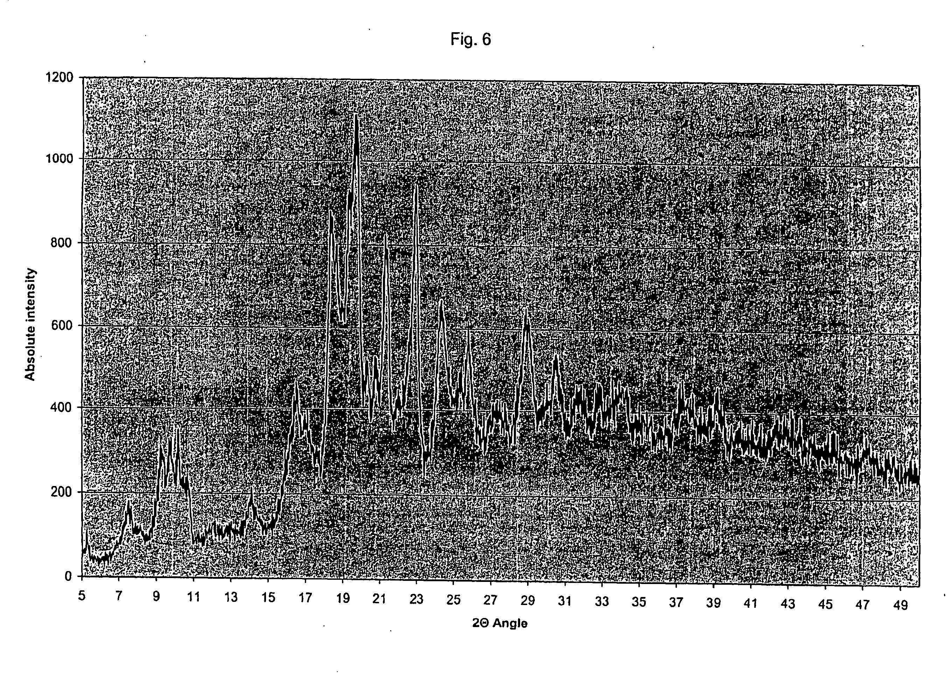

Potassium Atorvastatin, polymorph Form I (188.3 g; 0.32 mol) was recrystallised from 2-propanol. The resulting precipitate was isolated by filtration and washed on the filter with 2-propanol. The crystals were dried *in vacuo* at 45 °C. The material was analysed by HPLC analysis, which gave a purity of 100 % using the same HPLC analysis data as in example 1. The melting point by DSC analysis was 143-156 °C (See Fig. 6).

The X-ray powder diffractogram, obtained as in Example 1 above and presented in Fig. 6, showed a crystalline solid. Said pattern shows characteristic peaks at 2θ values of 18.44, 19.74 and 22.98. More particularly, polymorph Form III showed the following characteristic 2θ angle values:

| **2**Θ **angle** |
|---|
| 7.64 |
| 9.26 |
| 9.76 |
| 10.14 |
| 14.1 |
| 16.5 |
| 17.1 |
| **18.44** |
| **19.74** |
| 20.32 |
| 20.82 |
| 21.32 |
| **22.98** |
| 24.34 |
| 25.82 |
| 27.34 |
| 28.84 |
| 30.56 |
| 37.32 |
| 39.2 |

### Example 4

The above disclosed novel polymorphs I, II and III were tested for solubility characteristics in a range of solvents and pH values in comparison with the calcium salt of Atorvastatin and Atorvastatin as the free acid.

The tests were performed as follows:
100 mg of material was added to 100 ml of solvent.

After stirring for 10 min. the solution was visually inspected.

If all material was dissolved, an extra 50 mg of material was added followed by stirring for 10 min.

Another visual inspection was performed as described above. If the material was dissolved, the procedure was repeated until turbidity was observed. This was recorded as the solubility of the material.

If the material was undissolved, another 50ml of solvent was added, followed by stirring for 10 min. Another visual inspection was performed as described above. If the material was undissolved, the procedure was repeated until turbidity was no longer observed. This was recorded as the solubility of the material.

The results appear from Table I below.

**Table I**

| Solubility of different salts of atorvastatin and the free acid | | | | | |
|---|---|---|---|---|---|
| | Ca⁺⁺ salt | Free acid | K⁺ salt | | |
| Batch no | | | Form I | Form II | Form III |
| Water | Practically insoluble (< 1 g /10,000 ml) | Practically insoluble (< 1 g /10,000 ml) | Slightly soluble (1 g/670 ml) | Slightly soluble (1 g/700 ml) | Slightly soluble (1 g/670 ml) |
| 0.1 N HCl | Practically insoluble (< 1 g /10,000 ml) | Practically insoluble (< 1 g /10,000 ml) | Practically insoluble (< 1 g /10,000 ml) | Practically insoluble (< 1 g /10,000 ml) | Practically insoluble (< 1 g /10,000 ml) |
| H 4.0 | Practically insoluble (< 1 g /10,000 ml) | Practically insoluble (< 1 g /10,000 ml) | Slightly soluble (1 g /1000 ml) opalescent | Slightly soluble (1 g /1000 ml) opalescent | Slightly soluble (1 g /1000 ml) opalescent |
| pH 6.8 | Very slightly Soluble (1 g /10,000 ml) | Practically insoluble (< 1 g /10,000 ml) | Very slightly Soluble (1 g /2500 ml) | Very slightly Soluble (1 g /2500 ml) | Very slightly Soluble (1 g /2500 ml) |
| Ethanol | Very slightly Soluble (1 g /10,000 ml) | Freely soluble (1 g/6.7 ml) | Soluble (1 g/15 ml) | Freely soluble (1 g/ 6.7 ml) | Sparingly soluble (1 g/50 ml) |
| Methanol | Soluble (1 g/30 ml) | Freely soluble (1 g/5 ml) | Freely soluble (1 g/6.7 ml) | Freely soluble (1 g/5 ml) | Soluble (1 g/30 ml) |

## Claims

1. A novel polymorph Form I of the potassium salt of Atorvastatin, (βR,δR)-2-(p-fluorophenyl)-β,δ-dihydroxy-5-isopropyl-3-phenyl-4-(phenylcarbamoyl)pyrrole-1-heptanoic acid, **characterised in that** it is a white crystalline solid with a melting point of 155-165 °C, and having an X-ray powder diffraction pattern containing the following 2θ values measured using CuK_{α}-radiation: 19.92, 20.98 and 22.08.

2. The polymorph Form I of the potassium salt of Atorvastatin according to claim 1, wherein the X-ray powder diffraction pattern contains the following 2θ values measured using CuK_{α}-radiation:
| **2**Θ **angle** |
|---|
| 8.4 |
| 9.0 |
| 10.0 |
| 10.5 |
| 11.24 |
| 16.38 |
| 17.46 |
| 18.12 |
| **19.92** |
| **20.98** |
| **22.08** |
| 23.24 |
| 23.84 |
| 25.2 |
| 27.8 |
| 29.6 |
| 31.3 |
| 32.16 |
| 36.34 |
| 42.8 |

3. A novel polymorph Form II of the potassium salt of Atorvastatin, (βR,δR)-2-p-fluorophenyl)-β,δ-dihydroxy-5-isopropyl-3-phenyl-4-(phenylcarbamoyl)pyrrole-1-heptanoic acid, **characterised in that** it is a white amorphous solid with a melting point of 173-183°C.

4. A polymorph Form II of the potassium salt of Atorvastatin according to claim 3, having an X-ray powder diffraction pattern as illustrated in Fig. 5 of the drawings.

5. A novel polymorph Form III of the potassium salt of Atorvastatin, (βR,δR)-2-(p-fluorophenyl)-β,δ-dihydroxy-5-isopropyl-3-phenyl-4-(phenylcarbamoyl)pyrrole-1-heptanoic acid, **characterised in that** it is a white crystalline solid with a melting point of 143-156°C and having an X-ray powder diffraction pattern containing the following 2θ values measured using CuK_{α}-radiation: 18.44, 19.74 and 22.98.

6. The polymorph Form III of the potassium salt of Atorvastatin according to claim 5, wherein the X-ray powder diffraction pattern contains the following 2θ values measured using CuK_{α}-radiation:
| **2**Θ **angle** |
|---|
| 7.64 |
| 9.26 |
| 9.76 |
| 10.14 |
| 14.1 |
| 16.5 |
| 17.1 |
| **18.44** |
| **19.74** |
| 20.32 |
| 20.82 |
| 21.32 |
| **22.98** |
| 24.34 |
| 25.82 |
| 27.34 |
| 28.84 |
| 30.56 |
| 37.32 |
| 39.2 |

7. A method of preparing polymorph Form I of the potassium salt of Atorvastatin, (βR,δR)-2-(p-fluorophenyl)-β,δ-dihydroxy-5-isopropyl-3-phenyl-4-(phenylcarbamoyl)pyrrole-1-heptanoic acid according to claim 1, **characterised by** comprising the following steps: i) dissolution of Atorvastatin free acid in ethanol, ii) addition of potassium hydroxide, and iii) isolation of the precipitated potassium Atorvastatin polymorph Form I obtained by filtration.

8. A method of preparing polymorph Form II of the potassium salt of Atorvastatin, (βR,δR)-2-(p-fluorophenyl)-β,δ-dihydroxy-5-isopropyl-3-phenyl-4-(phenylcarbamoyl)pyrrole-1-heptanoic acid according to claim 3, **characterised by** comprising the following steps: i) dissolution of Atorvastatin free acid in 1-propanol, ii) addition of potassium hydroxide, and iii) isolation of the precipitated potassium Atorvastatin polymorph Form II obtained by filtration.

9. A method of preparing polymorph Form III of the potassium salt of Atorvastatin, (βR,δR)-2-(p-fluorophenyl)-β,δ-dihydroxy-5-isopropyl-3-phenyl-4-(phenylcarbamoyl)pyrrole-1-heptanoic acid according to claim 5, **characterised by** comprising the following steps: i) providing potassium Atorvastatin polymorph Form I, ii) dissolution thereof in 2-propanol, and iii) isolation of the precipitated potassium Atorvastatin Form III obtained by filtration.

10. A pharmaceutical formulation comprising as active ingredient a member selected from potassium Atorvastatin, Form I, potassium Atorvastatin, Form II and potassium Atorvastatin, Form III according to any of the claims 1-6, together with conventional pharmaceutically acceptable excipients, diluents, carriers and/or additives.

11. A pharmaceutical formulation according to claim 10 in the form of tablets, pills, dispersible granules, cachets, capsules, powders, lozenges, suppositories or retention enemas.

12. Use of a member selected among potassium Atorvastatin Form I, II or III according to any of the claims 1-6 for the preparation of a pharmaceutical formulation useful for treating hyper-cholesteremia or hyperlipidemia.

## Patentansprüche

1. Neue polymorphe Form I des Kaliumsalzes von Atorvastatin, (βR, δR)-2-(p-fluorphenyl)-β,δ-dihydroxy-5-isopropyl-3-phenyl-4-(phenylcarbamoyl)pyrrol-1-heptansäure, **dadurch gekennzeichnet, dass** es ein weißer kristalliner Feststoff mit einem Schmelzpunkt von 155-165 °C ist und ein Röntgenstrahlpulverdiffraktionsmuster aufweist, das die folgenden 20-Werte bei Messung unter Verwendung von CuK_{α}-Strahlung enthält: 19,92, 20,98 und 22,08.

2. Polymorphe Form I des Kaliumsalzes von Atorvastatin nach Anspruch 1, worin das Röntgenstrahlpulverdiffraktionsmuster die folgenden 20-Werte bei Messung unter Verwendung von CuK_{α}-Strahlung enthält:
| **2**Θ-**Winkel** |
|---|
| 8,4 |
| 9,0 |
| 10,0 |
| 10,5 |
| 11,24 |
| 16,38 |
| 17,46 |
| 18,12 |
| **19,92** |
| **20,98** |
| **22,08** |
| 23,24 |
| 23,84 |
| 25,2 |
| 27,8 |
| 29,6 |
| 31,3 |
| 32,16 |
| 36,34 |
| 42,8 |

3. Neue polymorphe Form II des Kaliumsalzes von Atorvastatin, (βR, δR)-2-(p-fluorphenyl)-β,δ-dihydroxy-5-isopropyl-3-phenyl-4-(phenylcarbamoyl)pyrrol-1-heptansäure, **dadurch gekennzeichnet, dass** es ein weißer amorpher Feststoff mit einem Schmelzpunkt von 173-183 °C ist.

4. Polymorphe Form II des Kaliumsalzes von Atorvastatin nach Anspruch 3 mit einem Röntgenstrahlpulverdiffraktionsmuster wie in der Abbildung von Figur 5 dargestellt.

5. Neue polymorphe Form II des Kaliumsalzes von Atorvastatin, (βR, δR)-2-(p-fluorphenyl)-β,δ-dihydroxy-5-isopropyl-3-phenyl-4-(phenylcarbamoyl)pyrrol-1-heptansäure, **dadurch gekennzeichnet, dass** es ein weißer kristalliner Feststoff mit einem Schmelzpunkt von 143-156 °C ist und ein Röntgenstrahlpulverdiffraktionsmuster aufweist, das die folgenden 20-Werte bei Messung unter Verwendung von CuK_{α}-Strahlung enthält: 18,44, 19,74 und 22,98.

6. Polymorphe Form III des Kaliumsalzes von Atorvastatin nach Anspruch 5 worin das Röntgenstrahlpulverdiffraktionsmuster die folgenden 20-Werte bei Messung unter Verwendung von CuK_{α}-Strahlung enthält:
| **2**Θ-**Winkel** |
|---|
| 7,64 |
| 9,26 |
| 9,76 |
| 10,14 |
| 14,1 |
| 16,5 |
| 17,1 |
| **18,44** |
| **19,74** |
| 20,32 |
| 20,82 |
| 21,32 |
| **22,98** |
| 24,34 |
| 25,82 |
| 27,34 |
| 28,84 |
| 30,56 |
| 37,32 |
| 39,2 |

7. Verfahren zum Herstellen der polymorphen Form I des Kaliumsalzes von Atorvastatin, (βR, δR)-2-(p-fluorphenyl)-β,δ-dihydroxy-5-isopropyl-3-phenyl-4-(phenylcarbamoyl)pyrrol-1-heptansäure nach Anspruch 1, **dadurch gekennzeichnet, dass** die folgenden Schritte umfasst sind: i) Lösen von freier Atorvastatin-Säure in Ethanol, ii) Zugabe von Kaliumhydroxid und iii) Isolierung der präzipitierten polymorphen Form I von Kalium-Atorvastatin, die durch Filtration erhalten wird.

8. Verfahren zum Herstellen der polymorphen Form II des Kaliumsalzes von Atorvastatin, (βR, δR)-2-(p-fluorphenyl)-β,δ-dihydroxy-5-isopropyl-3-phenyl-4-(phenylcarbamoyl)pyrrol-1-heptansäure nach Anspruch 3, **dadurch gekennzeichnet, dass** die folgenden Schritte umfasst sind: i) Lösen von freier Atovastin-Säure in 1-Propanol, ii) Zugabe von Kaliumhydroxid und iii) Isolierung der präzipitierten polymorphen Form II von Kalium-Atorvastatin, die durch Filtration erhalten wird.

9. Verfahren zum Herstellen der polymorphen Form III des Kaliumsalzes von Atorvastatin, (βR, δR)-2-(p-fluorphenyl)-β,δ-dihydroxy-5-isopropyl-3-phenyl-4-(phenylcarbamoyl)pyrrol-1-heptansäure nach Anspruch 5, **dadurch gekennzeichnet, dass** die folgenden Schritte umfasst sind: i) Bereitstellen der polymorphen Form I von Kalium-Atorvastatin, ii) Lösen dieser in 2-Propanol und iii) Isolierung der präzipitierten polymorphen Form III von Kalium-Atorvastatin, die durch Filtration erhalten wird.

10. Pharmazeutische Formulierung, umfassend als Wirkstoff ein Element, ausgewählt aus Kalium-Atorvastatin, Form I, Kalium-Atorvastatin, Form II, und Kalium-Atorvastatin, Form III, nach einem der Ansprüche 1-6, zusammen mit herkömmlichen pharmazeutisch verträglichen Exzipienten, Verdünnungsmitteln, Trägern und/oder Additiven.

11. Pharmazeutische Formulierung nach Anspruch 10 in der Form von Tabletten, Pillen, dispergierbaren Granalien, Kapseln aus Stärkemasse, Kapseln, Pulver, Lutschtabletten, Suppositorien oder Retentionseinläufen.

12. Verwendung eines Elements, ausgewählt aus Kalium-Atorvastatin Form I, II oder III, nach einem der Ansprüche 1-6 zur Herstellung einer pharmazeutischen Formulierung, die geeignet ist zum Behandeln von Hypercholersterolämie oder Hyperlipidämie.

## Revendications

1. Nouvelle forme polymorphe I du sel de potassium d'Atorvastatine, acide (βR, δR)-2-(p-fluorophényl)-β,δ-dihydroxy-5-isopropyl-3-phényl-4-(phénylcarbamoyl)pyrrole-1-heptanoïque, **caractérisée en ce qu'**elle est une matière solide cristalline blanche avec un point de fusion de 155-165°C et présentant un motif de diffraction de poudre aux rayons X contenant les valeurs 2θ suivantes mesurées en utilisant un rayonnement CuK_{α} : 19,92, 20,98 et 22,08.

2. Forme polymorphe I du sel de potassium d'Atorvastatine selon la revendication 1, dans laquelle le motif de diffraction de poudre aux rayons X contient les valeurs 2θ suivantes mesurées en utilisant un rayonnement CuK_{α} :
| Angle 2θ |
|---|
| 8,4 |
| 9,0 |
| 10,0 |
| 10,5 |
| 11,24 |
| 16,38 |
| 17,46 |
| 18,12 |
| 19,92 |
| 20,98 |
| 22,08 |
| 23,24 |
| 23,84 |
| 25,2 |
| 27,8 |
| 29,6 |
| 31,3 |
| 32,16 |
| 36,34 |
| 42,8 |

3. Nouvelle forme polymorphe II du sel de potassium d'Atorvastatine, acide (βR, δR)-2-(p-fluorophényl)-β,δ-dihydroxy-5-isopropyl-3-phényl-4-(phénylcarbamoyl)pyrrole-1-heptanoïque, **caractérisée en ce qu'**elle est une matière solide amorphe blanche avec un point de fusion de 173-183°C.

4. Forme polymorphe II du sel de potassium d'Atorvastatine selon la revendication 3 présentant un motif de diffraction de poudre aux rayons X comme illustré dans la figure 5 des dessins.

5. Nouvelle forme polymorphe III du sel de potassium d'Atorvastatine, acide (βR, δR)-2-(p-fluorophényl)-β,δ-dihydroxy-5-isopropyl-3-phényl-4-(phénylcarbamoyl)pyrrole-1-heptanoïque, **caractérisée en ce qu'**elle est une matière solide cristalline blanche avec un point de fusion de 143-156°C et présentant un motif de diffraction de poudre aux rayons X contenant les valeurs 2θ suivantes mesurées en utilisant un rayonnement CuK_{α} : 18,44, 19,74 et 22,98.

6. Forme polymorphe III du sel de potassium d'Atorvastatine selon la revendication 5, dans laquelle le motif de diffraction de poudre aux rayons X contient les valeurs 2θ suivantes mesurées en utilisant un rayonnement CuK_{α} :
| Angle 2θ |
|---|
| 7,64 |
| 9,26 |
| 9,76 |
| 10,14 |
| 14,1 |
| 16,5 |
| 17,1 |
| 18,44 |
| 19,74 |
| 20,32 |
| 20,82 |
| 21,32 |
| 22,98 |
| 24,34 |
| 25,82 |
| 27,34 |
| 28,84 |
| 30,56 |
| 37,32 |
| 39,2 |

7. Procédé de préparation d'une forme polymorphe I du sel de potassium d'Atorvastatine, acide (βR, δR)-2-(p-fluorophényl)-β,δ-dihydroxy-5-isopropyl-3-phényl-4-(phénylcarbamoyl)pyrrole-1-heptanoïque selon la revendication 1, **caractérisé en ce qu'**il comprend les étapes suivantes : i) de dissolution d'acide libre d'Atorvastatine dans de l'éthanol, ii) d'addition d'hydroxyde de potassium et iii) d'isolement de la forme polymorphe I de sel de potassium d'Atorvastatine précipitée obtenue par filtration.

8. Procédé de préparation d'une forme polymorphe I du sel de potassium d'Atorvastatine, acide (βR, δR)-2-(p-fluorophényl)-β,δ-dihydroxy-5-isopropyl-3-phényl-4-(phénylcarbamoyl)pyrrole-1-heptanoïque selon la revendication 3, **caractérisé en ce qu'**il comprend les étapes suivantes : i) de dissolution d'acide libre d'Atorvastatine dans du 1-propanol, ii) d'addition d'hydroxyde de potassium et iii) d'isolement de la forme polymorphe II de sel de potassium d'Atorvastatine précipitée obtenue par filtration.

9. Procédé de préparation d'une forme polymorphe III du sel de potassium d'Atorvastatine, acide (βR, δR)-2-(p-fluorophényl)-β,δ-dihydroxy-5-isopropyl-3-phényl-4-(phénylcarbamoyl)pyrrole-1-heptanoïque selon la revendication 5, **caractérisé en ce qu'**il comprend les étapes suivantes : i) de fourniture d'une forme polymorphe I de sel de potassium d'Atorvastatine, ii) de dissolution de celle-ci dans du 2-propanol et iii) d'isolement de la forme III de sel de potassium d'Atorvastatine précipitée obtenue par filtration.

10. Formulation pharmaceutique comprenant comme ingrédient actif un élément choisi parmi le sel de potassium d'Atorvastatine, forme I, le sel de potassium d'Atorvastatine, forme II et le sel de potassium d'Atorvastatine, forme III selon l'une quelconque des revendications 1-6 avec des excipients, des diluants, des supports et/ou des additifs classiques pharmaceutiquement acceptables.

11. Formulation pharmaceutique selon la revendication 10 dans la forme de boulettes, de pilules, de granulés dispersibles, de cachets, de gélules, de poudres, de pastilles, de suppositoires ou de lavements à garder.

12. Utilisation d'un élément choisi parmi le sel de potassium d'Atorvastatine forme I, II ou III selon l'une quelconque des revendications 1-6 pour la préparation d'une formulation pharmaceutique utile pour traiter l'hypercholestérolémie ou l'hyperlipidémie.
